# EUROPÄISCHE PATENTSCHRIFT

(11) **EP 2 348 851 B1**
(45) Veröffentlichungstag und Bekanntmachung des Hinweises auf die Patenterteilung: **01.05.2013**
(21) Anmeldenummer: 09796294.8
(22) Anmeldetag: 28.10.2009
(51) Int. Cl.: A01N 43/16

(54) **GLUCOSYLGLYCEROL ENTHALTENDE ZUSAMMENSETZUNG**
COMPOSITION CONTAINING GLUCOSYLGLYCEROL
COMPOSITION COMPRENANT DU GLUCOSYLGLYCÉROL

(30) Priorität: 28.10.2008 DE 102008053549
(43) Veröffentlichungstag der Anmeldung: 03.08.2011
(73) Patentinhaber: Bitop AG, 58453 Witten (DE)
(72) Erfinder: SCHWARZ, Thomas, 42799 Leichlingen (DE); KLEIN, Julia, 44869 Bochum (DE)
(74) Vertreter: Schöneborn, Holger
(86) Internationale Anmeldenummer: PCT/EP2009/007704
(87) Internationale Veröffentlichungsnummer: WO 2010/049136

(56) Entgegenhaltungen:
- EP-A1- 0 770 378
- WO-A1-2007/124991
- DATABASE WPI Week 200501 Thomson Scientific, London, GB; AN 2005-002845 XP002590142 & JP 2004 331577 A 25. November 2004 (2004-11-25)
- DATABASE WPI Week 199946 Thomson Scientific, London, GB; AN 1999-543849 XP002590143 & JP 11 222496 A 17. August 1999 (1999-08-17)

## Beschreibung

Die Erfindung betrifft eine Zusammensetzung auf Basis von Glucosylglycerol sowie ihre Verwendung.

Glucosylglycerol, genauer 2-O-α-D-Glucosylglycerol, ist als Naturstoff bekannt und wird beispielsweise von Cyanobakterien synthetisiert, denen es als osmoprotektive Substanz dient. Auf diese Weise gestattet es den Cyanobakterien das Wachstum in salzhaltigen Medien mit einer Konzentration von bis zu 1,5 M NaCl. Das Molekül wird in hohen Konzentrationen im Cytoplasma akkumuliert, um auf diese Weise den aufgrund der hohen Salzkonzentration in der Umgebung vorliegenden osmotischen Druck zu verringern und die Zelle vor Wasserverlust zu schützen. Ein Beispiel ist das Cyanobakterium *Synechocystis* sp. PCC 6803.

Darüber hinaus wird das Molekül auch von Pflanzen der Gattung Myrothamnus gebildet. Bei diesen Pflanzen handelt es sich um wechselfeuchte Pflanzen. *Myrothamnus flabellifolia* ist ein kleiner Strauch aus dem südlichen Afrika, der auf Felsplatten wächst und bis zu 60 cm hoch wird. Die im südlichen Afrika auftretenden mehrere Monate andauernden Dürreperioden übersteht die Pflanze unbeschadet in ausgetrocknetem Zustand. Sobald es jedoch regnet, wird die Pflanze innerhalb weniger Stunden wieder grün, woraus sich auch der Beiname "Auferstehungspftanze" ergibt. Die Struktur von 2-O-α-D-Glucosylglycerol ist wie folgt:

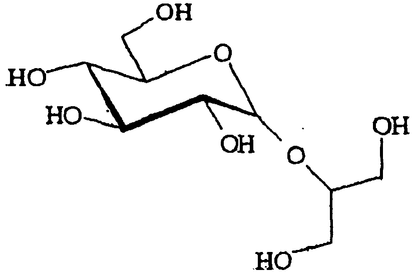

Die Verwendung von Glucosylglyceriden wie Glucosylglycerol für kosmetische und dermatologische Zubereitungen wird beispielsweise in der DE 195 40 749 A1 beschrieben. Die Substanz lässt sich z.B. als Moisturizer, d.h. als feuchtigkeitsspendende Substanz einsetzen. Daneben wurde bereits die Verwendung von Glucosylglycerol in Nahrungsmitteln, als Stabilisator von Biomolekülen und Mikroganismen, als Medikament für die Krebstherapie oder antibakterielles Mittel, z.B. in der JP 2004-331577, beschrieben.

Glucosylglycerol kann sowohl auf chemischem als auch auf enzymatischem Wege hergestellt werden. Die Synthese des natürlichen 2-O-α-D-Glucosylglycerols in hinsichtlich der Stereochemie reiner Form ist jedoch auf chemischem Wege aufgrund der geringen Ausbeuten praktisch nicht durchführbar. Die mikrobielle Synthese konnte zwar gezeigt werden, die Ausbeuten sind jedoch gering. Eine enzymatische Synthese, die die Transglucosylierung mittels α-Glucosidase nutzt, weist als Hauptnachteil auf, dass der Prozess die falsche Regioselektivität aufweist, da das Enzym die Glucosylierung der primären anstelle der sekundären Hydroxygruppe des Glycerins bevorzugt. Entsprechend enthält das Produktgemisch bei Verwendung von α-Glucosidase lediglich 30 % des korrekten natürlichen Glucosylglycerols, so dass die Aufarbeitung zur Isolierung des Produkts aufwendig ist.

Ein besonders vorteilhaftes Verfahren .zur Herstellung von 2-O-α-D-Glucosylglycerol wird in der WO 2008/034158 A2 beschrieben. Hier lässt man eine Saccharose-Phosphorylase auf eine Mischung einwirken, die einen Glucosyl-Donor und Glycerin als Glucosyl-Akzeptor aufweist. Bevorzugt handelt es sich bei dem Glucosyl-Donor um Saccharose. Daneben sind grundsätzlich auch Glucose-1-phosphat und α-D-Glucose-1-fluorid verwendbar. Die Selektivität ist ebenso wie die Ausbeute (> 95 %) außerordentlich gut.

Bei der Umsetzung von Saccharose mit Glycerin in Gegenwart der Saccharose-Phosphorylase entsteht zunächst ein Glucosyl-Enzym-Intermediat unter Freisetzung von Fructose. Das Glucosyl-Enzym-Intermediat wird anschließend von einem Nucleophil, hier Glycerin, angegriffen, wobei das gewünschte 2-O-α-D-Glucosylglycerol entsteht und das Enzym wieder freigesetzt wird. Als Nebenreaktion tritt die Hydrolyse des Glucosyl-Enzym-Intermediats unter Entstehung von Glucose auf. Die Reinigung des Produkts erfolgt anschließend mittels Chromatographie.

Es hat sich allerdings herausgestellt, dass nach einer säulenchromatographischen Aufreinigung des Produkts stets gewisse Mengen an Zuckern, insbesondere der Mono- und Disaccharide Glucose, Fructose und Saccharose im Produkt verbleiben. Aus diesem Grund ergibt sich das Problem, dass die im Produkt verbleibenden Zucker als Nahrung für Bakterien oder Pilze dienen können, was die Haltbarkeit der Glucosylglycerolzusammensetzung herabsetzt. Auf der anderen Seite soll der Zusatz von Konservierungsmitteln, Bakteriziden oder Fungiziden im Hinblick auf die Verwendung im kosmetischen Bereich oder als Nahrungsmittelzusatz möglichst vermieden werden.

Es stellt sich daher die Aufgabe, eine Zusammensetzung basierend auf Glucosylglycerol zur Verfügung zu stellen, die kein erhöhtes Bakterienwachstum und somit eine erhöhte Haltbarkeit zeigt.

Diese Aufgabe wird erfindungsgemäß gelöst durch eine Zusammensetzung enthaltend 20 - 80 Gew.-% Glucosylglycerol und 0,5 - 20 Gew.-% eines oder mehrerer Mono- und/oder Disaccharide. Bei der Zusammensetzung handelt es sich vorzugsweise um eine wässrige Lösung.

Überraschend konnte gezeigt werden, dass eine Zusammensetzung, die Glucosylglycerol in erhöhter Konzentration von 20 - 80 Gew.-% aufweist, langzeitstabil ist und kein erhöhtes Bakterienwachstum aufweist. Trotz der Gegenwart von Mono- und/oder Disacchariden tritt kein erhöhtes Bakterien-oder Pilzwachstum auf, vielmehr wird die Anzahl der Kolonie bildenden Einheiten durch eine erfindungsgemäße Zusammensetzung sogar deutlich verringert.

Bevorzugt handelt es sich bei dem Glucosylglycerol um das natürlich vorkommende 2-O-α-D-Glucosylglycerol, wie es beispielsweise von Cyanobakterien der Gattung *Synechocystis* akkumuliert wird. Grundsätzlich kann eine entsprechende Wirkung jedoch auch bei β-glycosidischer Verknüpfung der Glucose mit dem Glycerinmolekül oder bei Verknüpfung der Glucose mit dem Glycerin in 1-Position erwartet werden. Im Einzelnen sind somit neben dem natürlichen Glucosylglycerol auch 1-O-α-Glucosylglycerol, 1-O-β-Glucosylglycerol und 2-O-β-Glucosylglycerol in der D- und L-Konfiguration denkbar.

Die überraschende bakterizide Wirkung der erfindungsgemäßen Zusammensetzung konnte bei der nachfolgend dargestellten Keimzahluntersuchung mit *E.coli* DSM 1576 unter Verwendung von wässrigen Lösungen mit unterschiedlichen Konzentrationen an 2-O-α-D-Glucosylglycerol sowie 5 Gew.-% Glucose folgendermaßen gezeigt werden:

| Glucosylglycerol (Gew.-%) | Kolonie bildende Einheiten (CFU/g) | | | | | |
|---|---|---|---|---|---|---|
| | 0 Tage | 2 Tage | 7 Tage | 14 Tage | 21 Tage | 28 Tage |
| 50 | 300.000 | 150.000 | < 1.000 | < 100 | < 100 | < 100 |
| 30 | 300.000 | 130.000 | < 1.000 | < 100 | < 100 | < 100 |
| 20 | 300.000 | 280.000 | 100.000 | 5.000 | < 100 | < 100 |
| 0 | 300.000 | 4.000.000 | 2.200.000 | 1.500.000 | 1.100.000 | 1.000.000 |

CFU = colony forming unit = Kolonie bildende Einheit

Wie man erkennt, wird die Anzahl der Kolonie bildenden Einheiten durch eine hohe Glucosylglycerolkonzentration innerhalb weniger Tage stark herabgesetzt, während bei Verwendung der Vergleichslösung (5 Gew.-% Glucose) zunächst ein starker Anstieg der Zahl der Bakterienkolonien zu beobachten ist. In hoher Konzentration zeigt Glucosylglycerol somit offenbar eine stark bakterizide Wirkung.

Darüber hinaus wurde ein entsprechender Test mit dem Schimmelpilz *Aspergillus niger* und dem Hefepilz *Candida albicans* durchgeführt, wobei eine wässrige Lösung mit 50 Gew.-% 2-O-α-D-Glucosylglycerol und 3,5 Gew.-% Glucose verwendet wurde.

| | Kolonie bildende Einheiten (CFU/g) | | | | | |
|---|---|---|---|---|---|---|
| | 0 Tage | 2 Tage | 7 Taqe | 14 Taqe | 21 Tage | 28 Taqe |
| *A. niger* | 750.000 | 350.000 | 200.000 | 170.000 | 160.000 | 12.000 |
| *C. albicans* | 750.000 | 320.000 | 200.000 | 190.000 | 120.000 | 45.000 |

Glucosylglycerol in hoher Konzentration zeigt somit nicht nur eine bakterizide, sondern auch eine fungizide Wirkung, weshalb die Erfindung auch die Verwendung der konzentrierten Glucosylglycerol-Zusammensetzung als Bakterizid oder Fungizid betrifft.

Vorzugsweise wird das Glucosylglycerol in der Zusammensetzung in einer Konzentration von 30 - 70, weiter bevorzugt in einer Konzentration von 40 - 60 und besonders bevorzugt von ca. 50 Gew.-% verwendet. Eine 50 %-ige Lösung zeigt eine hervorragende Langzeitstabilität.

Die Konzentration an Mono- und/oder Disacchariden beträgt typischerweise 1 - 10 Gew.-%, insbesondere 3 - 7 Gew.-%. Bei der oben beschriebenen Synthese fallen in erster Linie die bei der enzymatischen Umsetzung entstehende Fructose, das Nebenprodukt Glucose sowie das Edukt Saccharose an.

Bei der wässrigen Lösung handelt es sich um eine klare, farblose bis hellgelbe Flüssigkeit mit einem pH-Wert von 5,5 - 6,0. Neben den erwähnten Stoffen können weitere Stoffe enthalten sein, insbesondere Glycerin, das nicht vollständig umgesetzt wurde.

Die erfindungsgemäße Zusammensetzung kann als Bestandteil von Hautpflegemitteln, beispielsweise Moisturizern, eingesetzt werden. Möglich ist auch die Verwendung als Bestandteil von Süßungsmitteln für Diabetiker, von Nahrungsergänzungsmitteln oder zur Stabilisierung von Biomolekülen wie Proteinen und Lipiden.

Je nach Verwendungszweck kann die Zusammensetzung weitere Stoffe, beispielsweise kosmetische Hilfsstoffe enthalten, z.B. Trägermittel, Parfüme, Lösungsvermittler, Vitamine, Stabilisatoren, Substanzen zum Verhindern des Schäumens, Verdickungsmittel, Farbstoffe, oberflächenaktive Substanzen, Emulgatoren oder feucht haltende Substanzen. Grundsätzlich möglich ist auch der Zusatz von Konservierungsmitteln und Bakteriziden, auch wenn eine weitreichende Langzeitstabilität ohne derartige Zusätze gewährleistet ist.

Die erfindungsgemäße Zusammensetzung kann auch in mikroverkapselter Form eingesetzt werden. Die Mikroverkapselung von Flüssigkeiten ist dem Fachmann z. B. auf dem Gebiet der Pharmazie hinlänglich bekannt. Auf diese Weise kann die orale Verabreichbarkeit vereinfacht, die Verträglichkeit verbessert und bei Bedarf auch eine verzögerte Freisetzung erreicht werden.

## Patentansprüche

1. Zusammensetzung enthaltend 20 - 80 Gew.-% Glucosylglycerol und 0,5 - 20 Gew.-% eines oder mehrerer Mono- und/oder Disaccharide.

2. Zusammensetzung nach Anspruch 1, **dadurch gekennzeichnet, dass** die Zusammensetzung eine wässrige Lösung ist.

3. Zusammensetzung nach Anspruch 1 oder 2, **dadurch gekennzeichnet, dass** das Glucosylglycerol 2-O-α-D-Glucosylglycerol ist.

4. Zusammensetzung nach einem der Ansprüche 1 bis 3, **dadurch gekennzeichnet, dass** die Konzentration an Glucosylglycerol 30 - 70 Gew.-% beträgt.

5. Zusammensetzung nach Anspruch 4, **dadurch gekennzeichnet, dass** die Konzentration an Glucosylglycerol 40 - 60 Gew.-%, insbesondere ca. 50 Gew.-% beträgt.

6. Zusammensetzung nach einem der Ansprüche 1 bis 5, **dadurch gekennzeichnet, dass** die Konzentration an Mono- und/oder Disacchariden 1 - 10 Gew.-%, insbesondere 3 - 7 Gew.-% beträgt.

7. Zusammensetzung nach einem der Ansprüche 1 bis 6, **dadurch gekennzeichnet, dass** die Mono- oder Disaccharide Glucose, Fructose und/oder Saccharose sind.

8. Verwendung einer Zusammensetzung nach einem der Ansprüche 1 bis 7 als Bestandteil eines Hautpflegemittels.

9. Verwendung einer Zusammensetzung nach einem der Ansprüche 1 bis 7 zur Stabilisierung von Biomolekülen.

10. Verwendung einer Zusammensetzung nach einem der Ansprüche 1 bis 7 als Bestandteil eines Süßungsmittels.

11. Verwendung einer Zusammensetzung nach einem der Ansprüche 1 bis 7 als Bakterizid, mit der Maßgabe, dass die Verwendung nicht der therapeutischen Behandlung des menschlichen oder tierischen Körpers dient.

12. Verwendung einer Zusammensetzung enthaltend 20 - 80 Gew.-% Glucosylglycerol, insbesondere 2-O-α-D-Glucosylglycerol, als Fungizid, mit der Maßgabe, dass die Verwendung nicht der therapeutischen Behandlung des menschlichen oder tierischen Körpers dient.

13. Mikrokapseln enthaltend eine Zusammensetzung nach einem der Ansprüche 1 bis 7.

## Claims

1. Composition containing 20 to 80 % w/w of glucosylglycerol and 0.5 to 20 % w/w of one or several mono- and/or disaccharides.

2. Composition according to claim 1, **characterized in that** the composition is an aqueous solution.

3. Composition according to claim 1 or 2, **characterized in that** the glucosylglycerol is 2-O-α-D-glucosylglycerol.

4. Composition according to any of claims 1 to 3, **characterized in that** the concentration of glucosylglycerol ranges between 30 and 70 % w/w.

5. Composition according to claim 4, **characterized in that** the concentration of glucosylglycerol ranges between 40 and 60 % w/w, and in particular is approximately 50 % w/w.

6. Composition according to any of claims 1 to 5, **characterized in that** the concentration of mono- and/or disaccharides ranges between 1 and 10 % w/w, in particular between 3 and 7 % w/w.

7. Composition according to any of claims 1 to 6, **characterized in that** the mono- or disaccharides are glucose, fructose and/or saccharose.

8. Use of a composition according to any of claims 1 to 7 as an ingredient of a skin care agent.

9. Use of a composition according to any of claims 1 to 7 for the stabilization of biomolecules.

10. Use of a composition according to any of claims 1 to 7 as an ingredient of a sweetening agent.

11. Use of a composition according to any of claims 1 to 7 as bactericide, subject to the proviso that the use is not for therapeutic treatment of the human or animal body.

12. Use of a composition containing between 20 and 80 % w/w of glucosylglycerol, in particular 2-O-α-D-glucosylglycerol, as fungicide, subject to the proviso that the use is not for therapeutic treatment of the human or animal body.

13. Microcapsules containing a composition according to any of claims 1 to 7.

## Revendications

1. Composition comprenant 20 - 80 % en poids de glucosylglycérole et 0,5 - 20 % en poids d'un ou de plusieurs mono- et/ou disaccharides.

2. Composition selon la revendication 1, **caractérisée en ce que** la composition est une solution aqueuse.

3. Composition selon la revendication 1 ou 2, **caractérisée en ce que** le glycosylglycérole est le 2-O-a-D-glycosylglycérole.

4. Composition selon l'une des revendications 1 à 3, **caractérisée en ce que** la concentration en glycosylglycérole représente 30 - 70 % en poids.

5. Composition selon la revendication 4, **caractérisée en ce que** la concentration en glycosylglycérole représente 40 - 60 % en poids, en particulier 50 % en poids.

6. Composition selon l'une des revendications 1 à 5, **caractérisée en ce que** la concentration en mono- et/ou disaccharides représente 1 - 10 % en poids, en particulier 3 - 7 % en poids.

7. Composition selon l'une des revendiations 1 à 6, **caractérisée en ce que** les mono- ou disaccharides sont le glycose, le fructose et/ou le saccharose.

8. Utilisation d'une composition selon l'une des revendications 1 à 7 en tant que composant d'un produit de soins pour la peau.

9. Utilisation d'une composition selon l'une des revendications 1 à 7 pour la stabilisation de biomolécules.

10. Utilisation d'une composition selon l'une des revendications 1 à 7 en tant que composant d'un édulcorant.

11. Utilisation d'une composition selon l'une des revendications 1 à 7 comme bactéricide, à condition que l'utilisation ne sert pas au traitement thérapeutique du corps humain ou animal.

12. Utilisation d'une composition comprenant 20 - 80 % en poids de glycosylglycérole, en particulier de 2-O-a-D-glycosylglycérole, en tant que fongicide, à condition que l'utilisation ne sert pas au traitement thérapeutique du corps humain ou animal.

13. Microcapsules contenant une composition selon l'une des revendications 1 à 7.
